# EUROPÄISCHE PATENTSCHRIFT

(11) **EP 2 121 066 B1**
(45) Veröffentlichungstag und Bekanntmachung des Hinweises auf die Patenterteilung: **27.03.2013**
(21) Anmeldenummer: 08706018.2
(22) Anmeldetag: 12.02.2008
(51) Int. Cl.: A61L 31/02, A61F 2/90, C22C 23/04, B22F 3/22

(54) **GITTERTEIL AUS METALL UND VERFAHREN ZUR HERSTELLUNG EINES GITTERTEILES**
LATTICE PART MADE OF METAL, AND METHOD FOR THE PRODUCTION OF A LATTICE PART
ÉLÉMENT GRILLE EN MÉTAL ET PROCÉDÉ DE PRODUCTION D'UN ÉLÉMENT GRILLE

(30) Priorität: 19.02.2007 AT 2592007
(43) Veröffentlichungstag der Anmeldung: 25.11.2009
(73) Patentinhaber: Austrian Research Centers GmbH-ARC, 1220 Wien (AT)
(72) Erfinder: ZAUNER, Rudolf, A-2500 Baden (AT); RIEMELMOSER, Franz, 84359 Simbach (DE); BAMMER, Manfred, A-1220 Wien (AT)
(74) Vertreter: Wildhack & Jellinek
(86) Internationale Anmeldenummer: PCT/AT2008/000048
(87) Internationale Veröffentlichungsnummer: WO 2008/101265

(56) Entgegenhaltungen:
- EP-A- 0 221 570
- EP-A- 0 888 757
- EP-A- 1 632 255
- US-A- 5 972 027
- US-B1- 6 641 776
- US-B1- 6 725 901
- STAIGER M P ET AL: "Magnesium and its alloys as orthopedic biomaterials: a review" 20051124, Bd. 27, 24. November 2005 (2005-11-24), Seiten 1728-1734, XP002469482

## Beschreibung

Die Erfindung bezieht sich auf einen gemäß dem Oberbegriff des Anspruches 1.

Weiters umfasst die Erfindung ein Verfahren zur Herstellung eines Gitterteiles aus Metall oder einer Metallitegierung mit einer metallischen Knotenbindung der Gitterstäbe, welches Gitter eine Dicke von weniger als 1 mm bei einer Größe der Durchbruchflächen von weniger als. 50 mm² aufweist.

Die US-A-5 972 027 offenbart ein Verfahren zur Herstellung eines porösen Gitterteils ausgebildet als Stent durch "Powder Injection Molding" (PIM), wobei ein Metallpulver in den Formenhohlraum eingebracht und zu einem Teil gepresst wird. Das Teil wird aus der Form ausgebracht und gesintert. Danach erfolgt eine Endfertigung des metallischen Gitterteils. Weil der Stent als monolithischer Teil hergestellt wird, besitzen die Gitterstäbe und die Gitterknoten senkrecht zur Gitterfläche gleiche Dicke. Die Dicke des Stents und die Größe der Durchbruchflächen sind nicht explizit offenbart.

Grobe Gitterteile aus Metall mit großen Durchbruchflächen und erheblichen Gitterstabdicken werden zumeist durch Gießen von Flüssigmetall in Formeln, z.B. Sandformen im offenen Herdguss oder Formguss hergestellt. Auch ein Ausschneiden der Durchbruchflächen aus dicken Blechen ist bekannt und ist für eine Schaffung von schweren Gittern dem Stand der Technik zuzuordnen.

Für eine Herstellung von feinen, ebenflächigen, metallischen Gitterteilen sind Gießverfahren meist nicht anwendbar, weil Gütemängel im Gussstück, wie Ungänzen, fehlende Knotenbindungen und dgl., oft unvermeidbar sind. Aus diesen Gründen werden Gitter mit dünnen Stäben und geringen Durchbruchflachen zumeist als Drahtgeflecht erstellt.

Feine Drahtgitter mit kleinen Durchbruchflächen haben zumeist gleichgestaltete enge Maschenweiten, weil die Dicke der Drähte im Geflecht mit abwechselnder Oberflächenpositionierung den gegenseitigen Abstand derselben festlegen und fixieren.

In Diagonalrichtung eines Gitters bzw. in einem Winkel von etwa 45° ist ein Drahtgitterteil, insbesondere bei fehlender Festlegung der Teilenden in großen Grenzen leicht verschiebbar und wieder rückführbar, weil dabei keine wesentlichen, plastischen Verformungen des Werkstoffes der Gitterdrähte erforderlich sind bzw. auftreten.

Um feine Gitter auch bei einer Beanspruchung durch Kräfte in Diagonalrichtung zu den Drahtlagen zu stabilisieren, wurde schon versucht, die Berührungstellen der Gitterstäbe in den Knoten durch Schweißen oder Löten zu verbinden. Eine derartige Verbindung der Gitterknoten ist zwar prinzipiell möglich, erfordert jedoch zusätzliche Aufwendungen.

Weiters erbringen leichte Gitter mit dünnen Wandstärken und vergrößerten Maschenweiten bzw. Durchbruchflächen Probleme im Hinblick auf eine Stabilität des Teiles, einer gleichmäßigen und gleichbleibenden Distanzierung der Stäbe oder Drähte von einander bzw. einer gleichmäßigen Zwischenflächendimension.

Drahtgitterteile mit geringem Gewicht können daher bei einer geforderten, feinen, geometrischen Gestalt, insbesondere bei geringem Verhältnis von Stabdicke zu lichte Maschenweite oft die Gebrauchseigenschaften für ein gewünschtes Anforderungsprofil nicht erfüllen.

Hier will die Erfindung die Mängel beseitigen und setzt sich zur Aufgabe, ein Gitterteil aus Metall zu schaffen, welches großen Freiraum in dessen Gestaltung sowie in der Erstellung der Gebrauchseigenschaften besitzt, im Wesentlichen keine größe, rückblidbare Diagonalverschiebung des Gitters zulässt, jedoch plastisch verformbar ist und eine genaue, feine Dimensionierung aufweist.

Ziel der Erfindung ist weiters, ein Verfahren zur Herstellung eines Gitterteiles aus Metall der eingangs genannten Art anzugeben, mittels welchen das Teil ein gewünschtes Eigenschaftsprofil, vorzugsweise für eine Verwendung als Stent, Stent ähnliche Systeme und Endoprothesen für den Einsatz im humanmedizinischen und veterinärmedizinischen Bereich, besitzt.

Die Aufgabe wird erfindungsgemäß mit den Merkmalen des Kennzeichens des Anspruches 1.

Die mit der Erfindung erzielten Vorteile bestehen insbesondere darin, dass eine Knotenbindung des Gitters demselben eine verbesserte Stabilität in diagonaler Richtung zu den Stäben verleiht und durch eine Bildung aus Leichtmetall geringe Teilgewichte erbringt. In vorteilhafter Weise können derartige, ebenflächige Gitter mit geringen Dicken bei gewünscht großen Durchbruchsflächen mechanische Eigenschaften aufweisen, durch welche deren Einsetzbarkeit für neue Anwendungen mit besonderen Anförderungsprofilen ermöglicht ist.

Erfindungsgemäß ist dadurch eine invivo Anwendung von Gitterteilen im und am Körper von Menschen und Tieren günstig.

Geometrisch, aber auch im Hinblick auf eine stabile, gegebenenfalls über die Achse stellenweise Aufweitung des Gitterteiles kann es günstig sein, wenn das Teil eine Rohrform, insbesondere eine ungeteilte Rohrform besitzt. Derart wird bei geringem Teilgewicht eine hohe Stabilität des Teiles erreicht

Wenn, wie erfindungsgemäß nach einer bevorzugten Ausbildung das Gitter durch Urformen von Metall gemäß DIN 8580 hergestellt ist, kann eine Diagonalverschiebung der Gitterstäbe ohne bleibende Verformung derselben in den Knoten unterbunden werden, wobei eine bleibende Formgebung der Gitterfläche begünstigt ist. Derart können räumliche Gitterflächen, z.B. teilweise aufgeweitete Gitterrohre, entsprechend dem gewünschten Eigenschaftsprofil gefertigt sein.

Für rohrförmige Gitterteile ist es weiters besonders vorteilhaft, wenn die Gitterstäbe zur Rohrachse einen Winkel von ungleich 90° oder ungleich 0°, insbesondere einen Winkel von etwa 45°, aufweisen. Derart werden bei einem Aufweiten des Gitterrohres die Gitterstäbe im Wesentlichen bleibend torsionsverformt, wobei eine Streckung bzw. Stabdehnung und deshalb eine Bruchgefahr minimiert ist.

Wenn zumindest eine der Gitteroberflächen spanend bearbeitet ist, sind auch bei einem Urformen eines Metallgitters sehr geringe Gitterdicken herstellbar, wodurch wirtschaftlichverfahrenstechnische und gütemäßige Vorteile erreicht sind und auch die Gittereigenschafen eine gewünschte Stabilität aufweisen.

Mit Vorteil weist überdies ein feines Gitterrohr eine Wandstärke von großer 0.1 mm, Jedoch kleiner als 0.4 mm und einen Durchmesser von größer 1 mm, Jedoch kleiner als 4 mm auf. Für derartige Gitter sind in einem technischen Anwendungsfeld, aber auch in einer medizinischen, insbesondere in einer endoprothetischen Versorgung von Menschen und Tieren wichtige Möglichkeiten gegeben.

Ist, wie nach einer bevorzugten Ausführungsform das Gitter plastisch verformbar, insbesondere ein Gitterrohr im Durchmesser bleibend aufweitbar und besteht der Gitterwerkstoff aus einer vorgenannten Magnesium-Basislegierung, so kann das Teil in günstiger Weise als Stent, Stent ähnliche Systeme und Endoprothesen für den Einsatz im humanmedizinischen und veterinärmedizinischen Bereich, Anwendung finden, insbesondere in Blutgefäßen von Menschen und Tieren eingesetzt werden. Die Körperflüssigkeiten lösen dabei langsam zumindest den Oberflächenbereich des metallischen Stents unter Bildung von Kalzium-Hydroxyl-Apatit, einer knorpelähnlichen Masse, welche vorzügliche Stützfunktionen übernehmen kann.

Das weitere Ziel der Erfindung, ein Verfahren zur Herstellung eines Gitterteiles aus Leichtmetall oder einer Leichtmetalllegierung mit einer metallischen Knotenbindung der Gitterstäbe, welches Gitter eine Dicke von weniger als 1 mm bei einer Größe der Durchbruchflächen von weniger als 50 mm² aufweist, zu schaffen, wird dadurch erreicht, dass das Gitter durch Urformen gemäß DIN 8580 hergestellt wird, wobei in einem ersten Schritt mindestens ein Teil einer Form gebildet wird, worauf nach einem Fügen der Form
in einem zweiten Schritt ein zumindest teilweise Metall oder eine dgl. Legierung enthaltendes Vormaterial in den Formenhohlraum eingebracht oder auf ein profiliertes Formenteil aufgebracht und eine Urformung begründet werden, wonach
in einem dritten Schritt das Teil aus bzw. von der Form zumindest teilweise ausgebracht wird und
in einem vierten bzw. Folgeschritt eine Endfertigung des metallischen Gitterteiles erfolgt.

Die Vorteile des erfindungsgemäßen Verfahrens einer Gitterteilherstellung sind vielschichtig und sind in einem Erreichen günstiger Gitterteileigenschaften in einer vorteilhaften Erzeugungsart und in einer hohen Wirtschaftlichkeit zu sehen bzw. begründet. Ein Urformen des feinen Gitters aus Metall sichert eine gewünschte Stabilität der Gitterknoten und die Eigenschaften, die bei einer Verwendung bevorzugt sind. In diesem besonderen Fall einer Umformung hat es sich als vorteilhaft erwiesen, wenn in einem ersten Schritt mindestens ein Teil der Form gebildet wird, weil derart eine hohe Flexibilität des Herstellverfahrens erreicht wird. Nach einem Fügen der Form, die auch eine Stützung derselben für ein druckbeaufschlagtes Einbringen des Metalls beinhaltet, erfolgt in einem zweiten Schritt ein Einbringen des Vormaterials in den Formhohlraum, wobei auch ein fließfähiges Metall mit erstarrten Partikeln vorteilhaft einsetzbar ist.

Nach einer weiteren Verfahrensvariante kann auch auf einem Formteil, der wie vorher dargelegt erstellt wurde, ein Metall nach dem Flüssig- bzw. Teilflüssig-Metallsprühverfahren aufgebracht und verfestigt werden. Im nachfolgenden dritten Schritt kann gegebenenfalls das Teil aus der Form ausgebracht oder nur teilweise ausgeformt werden, wobei ein Formenteil eine Stützfunktion z.B. für eine spanabhebende Bearbeitung des Gitters aufweist. Im letzten Schritt erfolgt eine Endfertigung des metallischen Gitterteiles, welche eine vorgesehene Einsatzform und Güte sicherstellt.

Wenn im ersten Schritt des Herstellungsverfahrens die Bildung von mindestens einem Teil der Form durch Powder Injection Molding (PIM) erfolgt, kann eine besonders exakte Formengestaltung erreicht werden.

Dabei kann es günstig sein, wenn das im ersten Schritt mittels Powder Injection Molding (PIM) gefertigte Formenteil durch Erstellung eines Formstoffes mittels Mischens von Pulver, wie Keramikpulver, und Binder mit einem Granulieren des Formstoffes und Spritzgießen des Granulates in eine Form unter Ausbildung eines Grünlings, welcher zu einem Braunling weitergebildet werden kann und Sintern desselben zu einem Formenteil vorzugsweise mit einer Porosität von 75 bis 95 Vol.-% hergestellt wird.

Derart wird nicht nur die Festigkeit der Form bzw. des Formwerkstoffes günstig beeinflusst, sondern auch ein Ausbringen eines Formteiles gefördert.

Im Hinblick auf eine besonders hohe Güte der Gitterstäbe und insbesondere der Gitterknoten, aber auch des Gewichtes und der Einsetzbarkeit des Gitterteiles wegen, hat es sich als vorteilhaft erwiesen, wenn als Leichtmetall eine Magnesium-Basislegierung, enthaltend in Gew.-%

| | | | | |
|---|---|---|---|---|
| Zink (Zn) | 0.6 | bis 8.0, vorzugsweise | 0.8 | bis 6.2 |
| Zirkon (Zr) | | bis 2.0, vorzugsweise | | bis 1.0 |
| Mangan (Mn) | 0.02 | bis 0.8, vorzugsweise | 0.04 | bis 0.6 |
| Calzium (Ca) | | bis 1.2, vorzugsweise | | bis 1.0 |
| Antimon (Sb) | | bis 0.8, vorzugsweise | | bis 0.5 |
| Aluminium (Al) | | bis 0.8, vorzugsweise | | bis 0.5 |
| Silber (Ag) | 0.08 | bis 2.5, vorzugsweise | 0.1 | bis 2.0 |

und Verunreinigungen mit Magnesium als Rest eingesetzt wird.

Wenn dabei im zweiten Schritt, insbesondere durch Druckgießen, zumindest teilflüssiges Metall in den Formenhohlraum eingebracht wird, kann eine Schwindung bei der Metallerstarrung in günstiger Weise vermindert und die Mikrostruktur des Gitterteiles vorteilhaft verfeinert werden.

Es ist auch möglich und kann für gewisse Herstellungsarten eines Gitterteiles vorteilhaft sein, wenn im zweiten Schritt Vormaterial aus Metallpulver und Binder, insbesondere durch Spritzgießen bzw. durch Metal Injection Molding (MIM) in den Formenhohlraum eingebracht wird und im vierten Schritt der Herstellung die Endfertigung des Gitterteiles durch Dichtsintem erfolgt.

Für eine Herstellung von besonders feinen und dünnen Gitterteilen, welche für eine spanende Bearbeitung keine ausreichende Gestaltungsfestigkeit besitzen, kann im dritten Schritt das Teil derart teilweise aus bzw. von der Form ausgebracht werden, dass dieses auf einen Formenteil belassen und von diesem gestützt wird, wonach im vierten Schritt durch spanabhebende Bearbeitung zur Herstellung der gewünschten, genauen Abmessungen und durch anschließende Entfernung des Stützformenteiles, insbesondere durch Auslaugen oder durch chemisches Auflösen desselben, eine Endfertigung des metallischen Gitterteiles erfolgt.

In der Folge soll beispielhaft eine erfindungsgemäße Herstellmöglichkeit eines Gitterteiles nach der Erfindung dargelegt werden, wobei hilfsweise graphische Darstellungen dienen mögen.

Es zeigen
- Fig. 1: ein Kernteil einer Form
- Fig. 2: eine Form mit Kernteil
- Fig. 3: eine umgossene Form
- Fig. 4: ein Gitterteil an einem Formkern
- Fig. 5: ein Gitterteil

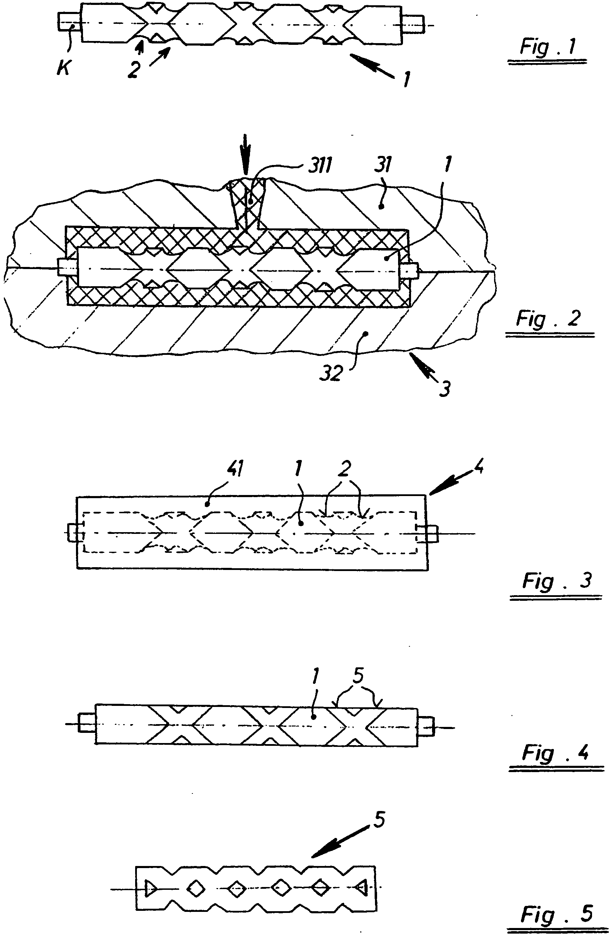

Durch ein Powder Injection Molding - Verfahren (PIM) wird gemäß Fig. 1 ein Kernteil 1 hergestellt und gesintert und weist Kemmarken K zu einer Halterung in einer Form und gitterbildende Vertiefungen 2 auf.

Fig. 2 zeigt einen Kernteil 1, eingesetzt in ein Spritzgusswerkzeug 3, gebildet durch ein Oberteil 31 mit einer Schmelzenzuführung 311 und einem Unterteil 32, wobei zwischen Kernteil 1 und Werkzeug 3 ein Formenhohlraum gebildet ist.

In Fig. 3 ist ein im Formenhohlraum erstarrter Gusskörper 4, nach einem Austrag eines Gusskörpers 4 aus einem Spritzgusswerkzeug 3, veranschaulicht, welcher einen Kernteil umgibt. Gitterbildende Vertiefungen 2 am Kernteil 1 sind ausgefüllt, wobei, wenn erforderlich, am Gusskörper 4 ein zylindrischer Außenteil 41, welcher gegebenenfalls für eine allseitige Bereitstellung von Vormaterial für ein Gitter dient, vorgesehen ist.

Nach einem spanenden Bearbeiten der Außenoberfläche eines Gusskörpers 4 am stützenden Kernteil 1 ist auf diesem ein Gitterteil 5 erstellt, wie in Fig. 4 dargestellt ist.

Wie in Fig. 5 dargestellt ist, ergibt sich nach einem chemischen Herauslösen eines Kernteiles 1 und gegebenenfalls eines weiteren Endbearbeitens ein Gitterteil 5 für eine vorgesehene Verwendung.

## Patentansprüche

1. Gitterteil aus Metall, **dadurch gekennzeichnet, dass** das Gitter eine Knotenbindung und eine Dicke von weniger als 1 mm, bei einer Größe der Durchbruchflächen von weniger als 50 mm², aufweist und die Gitterstäbe und die Gitterknoten senkrecht zur Gitterfläche gleiche Dicken haben, wobei das Gitterteil aus einer Magnesium-Basislegierung, enthaltend in Gew.-%
| | | | | |
|---|---|---|---|---|
| Zink (Zn) | 0.6 | bis 8.0, vorzugsweise | 0.8 | bis 6.2 |
| Zirkon (Zr) | | bis 2.0, vorzugsweise | | bis 1.0 |
| Mangan (Mn) | 0.02 | bis 0.8, vorzugsweise | 0.04 | bis 0.6 |
| Calzium (Ca) | | bis 1.2, vorzugsweise | | bis 1.0 |
| Antimon (Sb) | | bis 0.8, vorzugsweise | | bis 0.5 |
| Aluminium (Al) | | bis 0.8, vorzugsweise | | bis 0.5 |
| Silber (Ag) | 0.08 | bis 2.5, vorzugsweise | 0.1 | bis 2.0 |
und Verunreinigungen mit Magnesium als Rest besteht.

2. Gitterteil nach Anspruch 1, **dadurch gekennzeichnet, dass** das Teil eine Rohrform, insbesondere eine ungeteilte Rohrform aufweist.

3. Gitterteil nach Anspruch 1 oder 2, **dadurch gekennzeichnet, dass** das Gitter durch Urformen von Metall gemäß DIN 8580 hergestellt ist.

4. Gitterteil nach einem der Ansprüche 1 bis 3, **dadurch gekennzeichnet, dass** die Gitterstäbe zur Rohrachse einen Winkel von ungleich 90° oder ungleich 0°, insbesondere von etwa 45°, aufweisen.

5. Gitterteil nach einem der Ansprüche 1 bis 4, **dadurch gekennzeichnet, dass** zumindest eine der Gitteroberflächen spanabhebend bearbeitet ist.

6. Gitterteil nach einem der Ansprüche 1 bis 5, **dadurch gekennzeichnet, dass** das Gitterrohr eine Wandstärke von größer 0.1 mm, jedoch kleiner als 0,4 mm, und einen Durchmesser von größer 1 mm, jedoch kleiner als 4 mm, aufweist.

7. Gitterteil nach einem der Ansprüche 1 bis 6, **dadurch gekennzeichnet, dass** das Gitter plastisch verformbar, insbesondere ein Gitterrohr im Durchmesser bleibend, aufweitbar ist.

8. Verwendung eines Gitterteiles nach einem der Ansprüche 1 bis 7 als Stent, Stent ähnliche Systeme und Endoprothesen für den Einsatz im humanmedizinischen und veterinärmedizinischen Bereich.

9. Verfahren zur Herstellung eines Gitterteiles aus Metall oder einer Metalllegierung mit einer metallischen Knotenbindung der Gitterstäbe, welches Gitter eine Dicke von weniger als 1 mm bei einer Größe der Durchbruchflächen von weniger als 50 mm², aufweist, **dadurch gekennzeichnet, dass** das Gitter durch Urformen gemäß DIN 8580 von Leichtmetall, insbesondere einer Magnesium-Basislegierung, enthaltend in Gew.-%
| | | | | |
|---|---|---|---|---|
| Zink (Zn) | 0.6 | bis 8.0, vorzugsweise | 0.8 | bis 6.2 |
| Zirkon (Zr) | | bis 2.0, vorzugsweise | | bis 1.0 |
| Mangan (Mn) | 0.02 | bis 0.8, vorzugsweise | 0.04 | bis 0.6 |
| Calzium (Ca) | | bis 1.2, vorzugsweise | | bis 1.0 |
| Antimon (Sb) | | bis 0.8, vorzugsweise | | bis 0.5 |
| Aluminium (Al) | | bis 0.8, vorzugsweise | | bis 0.5 |
| Silber (Ag) | 0.08 | bis 2.5, vorzugsweise | 0.1 | bis 2.0 |
und Verunreinigungen mit Magnesium als Rest
hergestellt wird, wobei
in einem ersten Schritt mindestens ein Teil einer Form gebildet wird, worauf nach einem Fügen der Form
in einem zweiten Schritt ein zumindest teilweise Leichtmetall oder eine dgl. Legierung enthaltendes Vormaterial in den Formhohlraum eingebracht oder auf ein profiliertes Formteil aufgebracht und eine Urformung begründet werden, wonach in einem dritten Schritt das Teil aus bzw. von der Form zumindest teilweise ausgebracht wird und
in einem vierten bzw. Folgeschritt eine Endfertigung des metallischen Gitterteiles erfolgt.

10. Verfahren nach Anspruch 9, **dadurch gekennzeichnet, dass** im ersten Schritt die Bildung von mindestens einem Teil der Form durch "Powder Injection Molding" (PIM) erfolgt.

11. Verfahren nach Anspruch 9 oder 10, **dadurch gekennzeichnet, dass** im ersten Schritt das mittels "Powder Injection Molding" (PIM) gefertigte Formenteil durch Erstellung eines Formstoffes mittels Mischen von Pulver, wie Keramikpulver, und Binder mit einem Granulieren des Formstoffes und Spritzgießen des Granulates in eine Form unter Ausbildung eines Grünlings, welcher zu einem Braunling weitergebildet werden kann und Sintern desselben zu einem Formenteil, vorzugsweise mit einer Porosität von 75 bis 95 Vol.%, hergestellt wird.

12. Verfahren nach einem der Ansprüche 9 bis 11, **dadurch gekennzeichnet, dass** im zweiten Schritt, insbesondere durch Druckgießen, zumindest teilflüssiges Metall in den Formenhohlraum eingebracht wird.

13. Verfahren nach einem der Ansprüche 9 bis 12, **dadurch gekennzeichnet, dass** im zweiten Schritt Vormaterial aus Metallpulver und Binder, insbesondere durch Spritzgießen bzw. durch "Metall Injection Molding" (MIM) in den Formenhohlraum eingebracht wird und im vierten Schritt der Herstellung die Endfertigung des Gitterteiles durch Dichtsintem erfolgt.

14. Verfahren nach einem der Ansprüche 9 bis 13, **dadurch gekennzeichnet, dass** im dritten Schritt das Teil derart teilweise aus bzw. von der Form ausgebracht wird, dass dieses auf einem Formenteil belassen und von diesem gestützt wird, wonach im vierten Schritt durch spanabhebende Bearbeitung zur Erstellung der gewünschten, genauen Abmessungen und durch anschließende Entfernung des Stützformenteiles, insbesondere durch Auslaugen oder durch chemisches Auflösen desselben, eine Endfertigung des metallischen Gitterteiles erfolgt.

## Claims

1. A metal mesh element **characterised in that** said mesh has joint connections and a thickness of less than 1 mm, the size of the opening surfaces amounting to less than 50 mm², and **in that** the bars and joint connections having the same thickness in a direction perpendicular to the mesh surface, said mesh element consisting of a magnesium master alloy containing in per cent by weight
| | |
|---|---|
| zinc (Zn) | 0.6 to 8.0, preferably 0.8 to 6.2, |
| zirconium (Zr) | up to 2.0, preferably up to 1.0, |
| manganese (Mn) | 0.02 to 0.8, preferably 0.04 to 0.6, |
| calcium (Ca) | up to 1.2, preferably up to 1.0, |
| antimony (Sb) | up to 0.8, preferably up to 0.5, |
| aluminium (Al) | up to 0.8, preferably up to 0.5, |
| silver (Ag) | 0.08 to 2.5, preferably 0.1 to 2.0, |
the balance consisting of magnesium impurities.

2. The mesh element according to claim 1 characterise in that said element is tubular and particularly has the form of a one-piece tube.

3. The mesh element according to claim 1 or claim 2 **characterised in that** the mesh is produced by a primary metal shaping process according to the DIN 8580.

4. The mesh element according to any one of the claims 1 to 3 **characterised in that** the angle between the bars and the tube's axis is not 90° nor 0° and particularly approximately 45°.

5. The mesh element according to any one of the claims 1 to 4 **characterised in that** at least one of the mesh surfaces is subjected metal cutting.

6. The mesh element according to any one of the claims 1 to 5 **characterised in that** the mesh tube has a wall thickness of more than 0.1 mm, but less than 0.4 mm,
and a diameter of more than 1 mm, but less than 4 mm.

7. The mesh element according to any one of the claims 1 to 6 **characterised in that** the meshmay be plastically deformed and may be particularly expanded while maintaining the shape and diameter of a mesh tube.

8. The use of a mesh element according to any one of the claims 1 to 7 as a stent, a stent-like system or as an endoprosthesis for application in the field of human and veterinary medicine.

9. A process for producing a mesh element consisting of a metal or a metal alloy and having metal joint connections between its bars, said mesh having a thickness of less than 1 mm, the size of the opening surfaces amounting to less than 50 mm², **characterised in that** said mesh is produced from a light metal by a primary shaping process according to DIN 8580, said light metal particularly being a magnesium master alloy containing in per cent by weight
| | |
|---|---|
| zinc (Zn) | 0.6 to 8.0, preferably 0.8 to 6.2, |
| zirconium (Zr) | up to 2.0, preferably up to 1.0, |
| manganese (Mn) | 0.02 to 0.8, preferably 0.04 to 0.6, |
| calcium (Ca) | up to 1.2, preferably up to 1.0, |
| antimony (Sb) | up to 0.8, preferably up to 0.5, |
| aluminium (Al) | up to 0.8, preferably up to 0.5, |
| silver (Ag) | 0.08 to 2.5, preferably 0.1 to 2.0, |
the balance consisting of magnesium impurities,
at least one part of a mould being formed in a first step and, after assembling the mould, a primary material, which at least partially contains a light metal or a light alloy, being introduced into the mould's cavity or applied to a profiled part of the mould and a primary shaping process being initiated in a second step, after which the element is at least partially released from the mould in a third step and the processing of the metal mesh element is completed in a fourth/subsequent step.

10. The process according to claim 9 **characterised in that** said at least one part of the mould is formed by powder injection moulding (PIM) in the first step.

11. The process according to claim 9 or claim 10 **characterised in that** the part of the mould which is formed in the first step by powder injection moulding (PIM) is produced by obtaining a moulding material by mixing powders, such as ceramics powders, and binders and granulating said moulding material and injection moulding the obtained granulated material into a mould, forming a green compact, which can then be processed into a brown compact, said brown compact being sintered to obtain a part of a mould, preferably having a porosity of 75 to 95 °1° by volume.

12. The process according to any one of the claims 9 to 11 **characterised in that**, is the second step, at least partly liquid metal is introduced into the mould's cavity, particularly by means of diecasting.

13. The process according to any one of the claims 9 to 12 **characterised in that**, in the second step, primary material consisting of metal powders and binders is introduced into the mould's cavity, particularly by injection moulding or metal injection moulding (MIM), and **in that** the final processing of the mesh element consists in dense sintering it in the fourth step of the production process.

14. The process according to any one of the claims 9 to 13 **characterised in that**, in the third step the element is partially released from the mould in such a way that it remains on and is supported by one part of the mould, whereafter, in the fourth step, the processing of the metal mesh element is completed by metal cutting to obtain the desired exact dimensions and by subsequently removing the supporting part of the mould, particularly by leaching or chemically dissolving it.

## Revendications

1. Élément maillé métallique **caractérisé en ce que** les mailles présentent des jonctions et une épaisseur de moins de 1 mm, les aires ouvertes des mailles ayant moins de 50 mm², et **en ce que** l'épaisseur des barres et celle des jonctions des mailles, dans une direction perpendiculaire par rapport à la surface des mailles étant égales, ledit élément maillé consistant en un alliage de base de magnésium qui contient (en % en poids)
| | |
|---|---|
| zinc (Zn) | 0,6 à 8,0, de préférence 0,8 à 6,2, |
| zirconium (Zr) | jusqu'à 2,0, de préférence jusqu'à 1.0, |
| manganèse (Mn) | 0,02 à 0,8, de préférence 0,04 à 0,6, |
| calcium (Ca) | jusqu'à 1,2, de préférence jusqu'à 1,0, |
| antimoine (Sb) | jusqu'à 0.8, de préférence jusqu'à 0,5, |
| aluminium (Al) | jusqu'à 0.8, de préférence jusqu'à 0,5, |
| argent (Ag) | 0,8 à 2,5, de préférence 0,1 à 2,0, |
le reste étant des impuretés de magnésium.

2. Élément maillé selon la revendication 1, **caractérisé en ce que** ledit élément est tubulaire et notamment en forme d'un tube en une pièce.

3. Élément maillé selon la revendication 1 ou 2, **caractérisé en ce que** lesmaillessont produites par un procédé de formage primaire selon DIN 8580 d'un métal.

4. Élément maillé selon l'une des revendications 1 à 3, **caractérisé en ce que** l'angle entre les barres et l'axe du tube n'est ni 90° ni 0° et correspond notamment à environ 45°.

5. Élément maillé selon l'une des revendications 1 à 4, **caractérisé en ce qu'**au moins une surface des mailles est usinée par enlèvement des copeaux.

6. Élément maillé selon l'une des revendications 1 à 5, **caractérisé en ce que** l'épaisseur des parois du tube maillé est plus de 0,1 mm et moins de 0,4 mm et son diamètre est plus de 1 mm et moins de 4 mm.

7. Élément maillé selon l'une des revendications 1 à 6, **caractérisé en ce que** les mailles peuvent être déformées de manière plastique et peuvent notamment être évasées en gardant la forme et le diamètre d'un tube maillé.

8. Utilisation d'un élément maillé selon l'une des revendications 1 à 7 comme un stent, des systèmes similaires aux stents ou comme des endoprothèses pour l'utilisation en médicine humaine et vétérinaire.

9. Procédé pour produire un élément maillé consistant en un métal ou un alliage métallique avec des jonctions entre ses barres, l'épaisseur des mailles étant moins de 1 mm, les aires ouvertes étant moins de 50 mm², **caractérisé en ce que** les mailles sont produites d'un métal léger par un procédé de formage primaire selon DIN 8580, ledit métal léger étant notamment un alliage de base de magnésium qui contient (en % en poids)
| | |
|---|---|
| zinc (Zn) | 0,6 à 8,0, de préférence 0,8 à 6,2, |
| zirconium (Zr) | jusqu'à 2,0, de préférence jusqu'à 1,0, |
| manganèse (Mn) | 0,02 à 0,8, de préférence 0,04 à 0,6, |
| calcium (Ca) | jusqu'à 1,2, de préférence jusqu'à 1,0, |
| antimoine (Sb) | jusqu'à 0,8, de préférence jusqu'à 0,5, |
| aluminium (Al) | jusqu'à 0,8, de préférence jusqu'à 0,5, |
| argent (Ag) | 0,8 à 2,5, de préférence 0,1 à 2,0, |
le reste étant des impuretés de magnésium,
au moins une partie d'un moule étant formée dans une première étape et, après avoir assemblé le moule, dans une deuxième étape, une matière primaire, qui au moins partiellement contient un métal léger ou un alliage léger, étant introduite dans l'empreinte du moule ou appliquée sur une partie profilée du moule et un procédé de formage primaire étant lancé, après, dans une troisième étape, l'élément étant au moins partiellement démoulé et le traitement des mailles métalliques étant complété dans une quatrième étape/une étape suivante.

10. Procédé selon la revendication 9, **caractérisé en ce qu'**au moins une partie du moule est formée par moulage par injection de poudres (PIM, powder injection moulding).

11. Procédé selon la revendication 9 ou 10, **caractérisé en ce que** la partie du moule qui est formée dans la première étape par moulage par injection de poudres (PIM) est produite en utilisant une matière à mouler en mélangeant des poudres, comme, par exemple, des poudres céramiques, et des liants et en granulant ladite matière à mouler et introduisant la matière granulée dans un moule par moulage par injection, formant une pièce cruequi peut être traitée pour obtenir une pièce déliantée qui est ensuite frittée pour obtenir ladite partie du moule, de préférence avec une porosité de 75 à 95 % en volume.

12. Procédé selon l'une des revendications 9 à 11, **caractérisé en ce que** du métal qui est au moins partiellement liquide est introduite dans l'empreinte du moule dans la deuxième étape, notamment par un procédé de coulée sous pression.

13. Procédé selon l'une des revendications 9 à 12, **caractérisé en ce que** de la matière primaire consistant en des poudres métalliques et des liants est introduite dans l'empreinte du moule dans la deuxième étape, notamment par moulage par injection ou moulage par injection de métal (MIM, metal injection moulding) et **en ce que** la finition de l'élément maillé est un procédé de frittage à densité maximale dans la quatrième étape.

14. Procédé selon l'une des revendications 9 à 13, **caractérisé en ce que** l'élément est partiellement démoulé dans la troisième étape, ainsi qu'il reste sur et est supporté par une partie du moule, et après, dans la quatrième étape, le traitement de l'élément maillé métallique est fini par l'enlèvement des copeaux pour obtenir exactement les
dimensions désirées et par l'enlèvement ultérieur de la partie de support du moule, notamment par lixiviation ou dissolution chimique.
